# EUROPÄISCHE PATENTANMELDUNG

(11) **EP 0 790 037 A2**
(43) Veröffentlichungstag der Anmeldung: **20.08.1997**
(21) Anmeldenummer: 97100306.6
(22) Anmeldetag: 10.01.1997
(51) Int. Cl.: A61B 17/39

(54) **Bipolare Pinzette**

(30) Priorität: 14.02.1996 DE 29602593 U; 06.04.1996 DE 29606409 U
(71) Anmelder: S & T Marketing AG, CH-8212 Neuhausen (CH)
(72) Erfinder: Vogel, Max, CH 8252 Schlatt (CH); Tritt, Erich, D 79798 Jestetten (DE)
(74) Vertreter: Hiebsch, Gerhard F., Dipl.-Ing.

(57) **Zusammenfassung**

Bei einer bipolaren Pinzette mit zwei einends durch eine Halterung verbundenen sowie gegeneinander elektrisch isolierten Pinzettenarmen (12ₐ) weist die Halterung zwei miteinander verbundene rinnenartige Profile (24ₐ) auf, deren jedes mit zwei Einsteckschlitzen od.dgl. Führungsbahnen für jeweils einen blattartigen Bereich oder ein Griffblatt (20) eines der Pinzettenarme (12ₐ) versehen ist und einer Längskante des Pinzettenarmes bzw. Griffblattes anliegt. Die rinnenartigen Profile (24ₐ) sind aus keramischem Werkstoff oder aus metallischem Werkstoff geformt; letzterer ist mit einer elektrisch isolierenden Mantelschicht versehen.

## Beschreibung

Die Erfindung betrifft eine bipolare Pinzette mit zwei einends durch eine Halterung verbundenen sowie gegeneinander elektrisch isolierten Pinzettenarmen.

Koagulationspinzetten dieser Art mit Pinzettenarmen, die jeweils eine isolierende Ummantelung sowie eine aus dieser ragende Spitze aufweisen, offenbart die EP-A-0 092 170 der Anmelderin. Die Pinzettenschenkel oder -arme sind durch einen Oxidüberzug gegeneinander isoliert, welcher die Spitzen frei läßt. Beschrieben wird auch, daß im Prinzip die Ummantelung eines der beiden Pinzettenarme zur Isolierung ausreicht oder aber ein Oxidüberzug am spitzen fernen Ende der Pinzettenarme, welche dort an einem zwischen ihnen verlaufenden, querschnittlich I-förmigen Metallhalter festgelegt sind. Die Enden der Pinzettenarme ragen über den Metallhalter hinaus und sind mit Senkbohrungen für anzubringende Kontakte versehen.

Mit einer derartigen Pinzette kann -- insbesondere in der Mikrochirurgie -- eine an die beiden Pinzettenarme angelegte elektrische Hochfrequenzspannung beispielsweise zur Verödung kleiner Blutgefäße herangezogen werden. Insbesondere durch die außenliegenden Kontaktbohrungen in freien Endabschnitten der Pinzettenarme ist jedoch eine kontaktsichere, medizinischen Hygieneanforderungen genügende Stromversorgung technisch schwierig und aufwendig in der Realisierung.

Auch sind Koagulationspinzetten bekannt geworden, deren Pinzettenarme in einem Kunststoffkörper festgehalten werden. Der Einsatz solcher Koagulationspinzetten ist nur begrenzt möglich, da der Kunststoff nicht von ausreichender Hitzebeständigkeit bezüglich des erforderlichen Sterilisierens ist.

In Kenntnis dieses Standes der Technik hat sich der Erfinder das Ziel gesetzt, eine bipolare Pinzette der eingangs beschriebenen Art bezüglich ihrer Herstellung, Handhabung und ihren Einsatzmöglichkeiten weitergehend zu verbessern.

Zur Lösung dieser Aufgabe führt die Lehre des unabhängigen Anspruches; die Unteransprüche geben günstige Weiterbildungen an.

Erfindungsgemäß besteht die Halterung aus zwei miteinander verbundenen rinnenartigen Profilen, deren jedes mit zwei Einsteckschlitzen od.dgl. Führungsbahnen für jeweils einen blattartigen Bereich oder ein Griffblatt eines Pinzettenarmes versehen ist und in Gebrauchsstellung der Pinzette einer Längskante des Pinzettenarmes oder des Griffblattes anliegt; die beiden die Halterung ergebenden Rinnenprofile ergreifen also die beiden Pinzettenarme jeweils von einer Längskante und werden dabei von Schrauben od.dgl. Haltegliedern zusammengehalten.

Die Rinnenprofile sind in einer Ausführung aus Metall --bevorzugt einem stranggepreßten Leichtmetall -- und können zur Aufnahme nicht isolierter Pinzettenarme ihrerseits mit einem isolierenden Mantel versehen sein.

Als günstig hat es sich bei isolierten Pinzettenarmen erwiesen, an diesen einen deren Ummantelung durchgreifenden Kontaktstift für einen elektrischen Anschluß festzulegen, der das ihm zugeordnete Rinnenprofil axial überragt.

Dank dieser Maßgabe entsteht eine kompakte Pinzette mit einer Halterung, welche als Ansatz für ein elektrisches Steckelement dienen kann, das man über die beiden parallelen Kontaktstifte schiebt und somit auf einfache Weise den erforderlichen Strom in die Pinzettenarme einleitet.

Die Pinzettenarme einer Ausführung der erfindungsgemäßen Pinzette sind in an sich bekannter Weise oxidbeschichtete Metallstreifen mit eingeprägtem Griffteil; der Kontaktstift wird vor dem Flammspritzen des Oxidüberzuges am Metallkern verschweißt oder nach der Beschichtung angebracht; dann muß die Kontaktschicht an der Befestigungsstelle durchbrochen werden. Die Ummantelung der Metallstreifen kann z.B. aus Keramikwerkstoff gefertigt sein.

Von besonderer Bedeutung ist eine selbstisolierende Halterung aus Keramikwerkstoff. Bei dieser können die Pinzettenarme kopfseitig überstehen; ihre freien Enden sind dann Verlängerungsblätter mit stromführenden Kontaktflächen.

Beim erfindungsgemäßen Rinnenprofil aus metallischem oder keramischem Werkstoff erstreckt sich im Rinneninnenraum eine angeformte Rippe, deren Seitenwände zusammen mit den Innenflächen der Rinnenwände die erwähnten Einschubschlitze definieren.

Um den Klemmvorgang für die Pinzettenarme zu vereinfachen, können die Seitenflächen der Rippe konvex ausgebildet werden, so daß etwa in der Seitenmitte die engste Stelle des Einsteckschlitzes eine zur Pinzettenlängsachse parallele Gerade bildet.

Die Rippe endet nach einem Merkmal zu einer Ausgestaltung in Abstand zum freien Ende des Rinnenprofils und begrenzt so einen Kopfraum zur Aufnahme der an die Fläche der Pinzettenarme angefügten Kontaktstifte.

Die beiden Rinnenprofile werden -- wie gesagt -- durch Verbindungsglieder, insbesondere Schrauben, zusammengehalten, dank deren auch der Abstand zwischen dem Rinnenprofil eingestellt werden kann.

Im übrigen schlagen die Rinnenprofile des einfacheren Zusammenbaus halber gegebenenfalls an zwei seitlichen Schultern der Pinzettenarme an, diese Schultern sind in den Längskanten der Pinzettenarme vorgesehen und zwar an der Übergangsstelle zu dem erwähnten Griffblatt.

Bei den keramischen Rinnenprofilen hat es sich als günstig erwiesen, den zugeordneten Stromanschlußstecker mit zwei Einschubschlitzen für die genannten Verlängerungsblätter der Pinzette auszustatten; im Innenraum des erfindungsgemäß querschnittlich rechteckigen Stromanschlußsteckers begrenzt ein Klemmblock jene Einschubschlitze. Letzterer führt Kontaktadern als Partner der Kontaktflächen der Verlängerungsblätter.

Eine weitere Ausgestaltung des Erfindungsgegenstandes bietet im Bereich des freien Kopfraumes an den einander Zugewandten freien Innenflächen der Griffblätter einen von isolierendem Oxidmaterial freien Kontaktabschnitt an, an den ein -- gegebenenfalls federnder -- Kontaktstift od.dgl. Kontaktelement eines in den freien Kopfraum einführbaren Kontaktsteckers anlegbar ist. Letzterer kann lösbar im Kopfraum ruhen.

Auf die beschriebene Weise läßt sich besonders vorteilhaft eine optimal geschützte, kontaktsichere und insbesondere für den Einsatz unter hygienisch sensiblen Bedingungen optimierte Stromversorgung einer bipolaren Pinzette realisieren.

Insgesamt ergibt sich eine bipolar mit Strom beaufschlagbare Pinzette einfacher Gestalt, die allen Anforderungen --auch denen höherer Temperaturen -- genügt und problemlos zu handhaben ist.

Weitere Vorteile, Merkmale und Einzelheiten der Erfindung ergeben sich aus der nachfolgenden Beschreibung eines bevorzugten Ausführungsbeispiels sowie anhand der Zeichnung; diese zeigt in
- Fig. 1:: die Draufsicht auf eine auseinandergenommene Pinzette;
- Fig. 2:: ein gegenüber Fig. 1 vergrößertes, im Längsschnitt dargestelltes Rinnenprofil als Teil der Pinzette;
- Fig. 3:: die Draufsicht auf das Rinnenprofil;
- Fig. 4:: die teilweise geschnittene Draufsicht auf das freie Ende der Pinzette;
- Fig. 5:: das freie Ende der Pinzette in Schrägsicht;
- Fig. 6:: eine Schrägsicht auf einen Teil einer anderen Pinzette mit zugeordnetem Stecker.

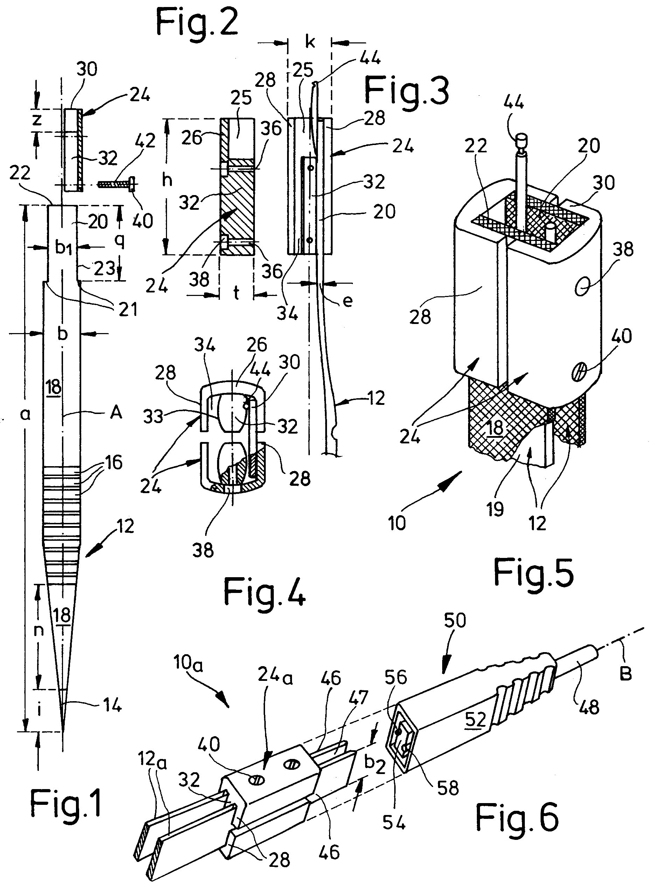

Eine Pinzette 10 der Gesamtlänge a von etwa 130 mm weist zwei -- einends miteinander parallel verbundene -- streifenartige Pinzettenarme 12 auf. Deren freies Armende verjüngt sich zu einer Spitze 14 der Länge i von etwa 10 mm, an die in einem Abstand n von etwa 27 mm ein -- von mehreren Querrillen 16 gebildeter -- Griffbereich anschließt; dort mißt die maximale Breite b des Pinzettenarms 12 etwa 9 mm. Die griffnahe Dicke e des Pinzettenarmes 12 beträgt etwa 0,8 mm; gemäß Fig. 3 nimmt diese Dicke e zum Griffbereich hin zu.

Der metallische Pinzettenarm 12 ist mit einer rauhen Oxidschicht aus elektrisch isolierendem Material ummantelt, was beispielsweise auf dem Wege des Plasmaspritzens mit Aluminiumoxid erfolgt. Aus dem -- in Fig. 5 bei 18 auf einem Metallkern 19 überhöht angedeuteten -- Oxidmantel ragt die blanke Spitze 14 hervor.

Das andere Ende des Pinzettenarmes 12 ist im Bereich eines Griffblattes 20 auf eine Länge q von etwa 20 mm zu einer konstanten Breite b₁ auf ein Maß von 6,5 mm querschnittlich verkürzt. Es entstehen so zwei schulterartige Absätze 21, von den einer auf jeder Seite der eine Symmetriegerade bildenden Längsachse A angeordnet ist. Durch diese Ausgestaltung entsteht ein spatelartiges Griffblatt 20 mit rechtwinkelig zur Längsachse A stehender Endkante 22.

Das Griffblatt 20 dient der Festlegung zweier Rinnenprofile 24 der Länge h von 20 mm aus Rinnenboden 26 der äußeren Breite k von 6 mm und parallelen Rinnenschenkeln 28 der äußeren Querschnittslänge t von 4 mm; diese werden gegenläufig mit sich zueinander öffnenden Rinnenräumen auf die Griffblattlängskanten 23 gesteckt.

In Abstand z (etwa 5 mm) zum spitzenfernen Profilrand 30 des Rinnenprofils 24 endet eine in diesem mittig angeformte Rippe 32, die mit den Rinnenschenkeln 28 jeweils einen Einsteckschlitz 34 für die Griffblätter 20 der beiden parallelen Pinzettenarme 12 begrenzt. Des besseren Preßsitzes halber sind die Rippenseiten 33 gemäß Fig. 4 querschnittlich nach außen teilellipsenförmig so gebogen, daß die engste Stelle des Einsteckschlitzes 34 etwa in der Mitte der Rippenseite 33 verläuft.

Außerdem ist eine der beiden Rippen 32 der einander erganzenden Rippenprofile 24 von zwei Gewindebohrungen 36 durchsetzt; damit fluchten in Endstellung der Pinzette 10 Bohrungen 36 der anderen Rippe 32, und diese münden jeweils in einer zylindrischen Erweiterung 38. Letztere nimmt den Kopf 40 einer Schraube 42 auf, welche die Rinnenprofile 24 zusammenhält und gegen die Griffblattlängskanten 23 drückt.

Das gesamte Rinnenprofil 24 ist aus einer Aluminiumlegierung hergestellt, bevorzugt stranggepreßt, und -- nach Ablängung des Abschnitts jener Länge z der Rippe 32 --eloxiert. Dank dieser Ablängung der Rippe 32 entsteht im Rinnenprofil 24 ein freier Kopfraum 25, in dem ein am jeweiligen Metallkern 19 im Bereich des Griffblattes 20 festgelegter und mit dem jeweiligen Metallkern 19 einen elektrischen Kontakt ausbildender Kontaktstift 44 verläuft.

Somit besteht die Pinzette 10 aus den beiden Pinzettenarmen 12 sowie dem sie haltenden und zusammengeschraubten Paar von Rinnenprofilen 24, die gleichzeitig eine Steckhalterung für einen -- zu dieser Ausführung in der Zeichnung nicht dargestellten -- Stromanschlußstecker anbieten; letzterer wird mit den erwähnten Kontaktstiften 44 verbunden und erlaubt einen Stromfluß durch den Metallkern 19 der beiden Pinzettenarme 12 zu deren Spitzen 14, die mit Strom unterschiedlicher Polarität beaufschlagt werden.

Sind die metallischen Rinnenprofile 24 mit einem isolierenden Werkstoff beschichtet, kann die Mantelschicht 18 auf den Pinzettenarmen entfallen.

Bei der Pinzette 10ₐ der Fig. 6 sind die Rinnenprofile 24ₐ aus einem Keramikwerkstoff geformt; diese Rinnenprofile 24ₐ halten -- wie die erwähnten Metallprofile mit isolierender Beschichtung -- die Pinzettenarme 12ₐ unterschiedlicher Polarität zusammen. Die Pinzettenarme 12a verlängernde beschichtungsfreie Verlängerungsblätter 46 einer Breite b₂ ersetzen hier die oben erörterten Kontaktstifte 44.

Auf die -- Kontaktflächen 47 anbietenden -- Verlängerungsblätter 46 wird ein an einem Kabelende 48 angebrachter Stromanschlußstecker 50 rechteckigen Querschnitts aufgeschoben, mit dessen Seitenwänden 52 ein axialer Klemmblock 54 Einschubschlitze 56 für die Verlängerungsblätter 46 begrenzt; in diesem Klemmblock 54 verlaufen parallel zur Kabelachse B Kontaktadern 58 zur Anlage an den Kontaktflächen 47.

## Patentansprüche

1. Bipolare Pinzette mit zwei einends durch eine Halterung verbundenen sowie gegeneinander elektrisch isolierten Pinzettenarmen,
dadurch gekennzeichnet,
daß die Halterung zwei miteinander verbundene rinnenartige Profile (24, 24ₐ) aufweist, deren jedes mit zwei Einsteckschlitzen (34) od.dgl. Führungsbahnen für jeweils einen blattartigen Bereich oder ein Griffblatt (20) eines der Pinzettenarme (12, 12ₐ) versehen ist und einer Längskante (23) des Pinzettenarmes bzw. Griffblattes anliegt.

2. Pinzette nach Anspruch 1, dadurch gekennzeichnet, daß die rinnenartigen Profile (24) aus metallischem Werkstoff geformt sind.

3. Pinzette nach Anspruch 1 oder 2, dadurch gekennzeichent, daß die rinnenartigen Profile (24) aus metallischem Werkstoff mit einer elektrisch isolierenden Mantelschicht versehen sind.

4. Pinzette nach Anspruch 1, dadurch gekennzeichnet, daß die rinnenartigen Profile (24ₐ) aus keramischem Werkstoff bestehen.

5. Pinzette mit jeweils eine isolierende Ummantelung und eine aus dieser ragende Spitze aufweisenden Pinzettenarmen sowie mit elektrischen Anschlußstellen an den der Halterung nahen Enden der Pinzettenarme nach Anspruch 1 oder 3, dadurch gekennzeichnet, daß am Pinzettenarm (12) ein dessen Ummantelung (18) durchgreifender Kontaktstift (44) für einen Stromanschlußstecker od.dgl. festgelegt ist, wobei gegebenenfalls zwei zur Längsachse (A) ihrer Pinzettenarme (12) parallele Kontaktstifte (44) vorhanden sind, welche die Rinnenprofile (24) axial überragen, und/oder die isolierende Ummantelung (18) aus Keramikwerkstoff besteht.

6. Pinzette mit elektrischen Anschlußstellen an den der Halterung nahen Enden der Pinzettenarme nach Anspruch 1, 3 oder 4, dadurch gekennzeichnet, daß am Pinzettenarm (12, 12ₐ) ein über die Rinnenprofile (24, 24ₐ) hinausragendes Verlängerungsblatt (46) mit zumindest einer Kontaktfläche (47) für einen Stromanschlußstecker (50) od.dgl. angeordnet ist.

7. Pinzette nach einem der Ansprüche 1 bis 6, dadurch gekennzeichnet, daß die beiden Einsteckschlitze (34) od.dgl. Führungsbahnen des Rinnenprofils (24, 24ₐ) durch dessen beide Rinnenschenkel (28) und eine zwischen diesen verlaufende Rippe (32) begrenzt sind, wobei gegebenenfalls die Rippe (32) an den Rinnenboden (26) des Rinnenprofils (24, 24ₐ) innenseitig angeformt und/oder wenigstens eine der den Einsteckschlitz (34) od.dgl. Führungsbahn begrenzenden Wandflächen teilweise zum Einsteckschlitz hin konvex gekrümmt ist.

8. Pinzette nach Anspruch 7, dadurch gekennzeichnet, daß die Rippe (32) seitlich querschnittlich teilelliptische sowie einwärts etwa konvergierende Konturen der Seitenflächen (33) aufweist, wobei gegebenenfalls die engste Querschnittsstelle des Einsteckschlitzes (34) od.dgl. Führungsbahn etwa in der Mitte der Seitenfläche (32) verläuft.

9. Pinzette nach einem der Ansprüche 1 bis 8, dadurch gekennzeichnet, daß die Rippe (32) in Abstand (z) zum freien Ende des Rinnenprofils (24) endet und einen Kopfraum (25) begrenzt, wobei gegebenenfalls im Kopfraum (25) ein Abschnitt der Kontaktstifte (44) verläuft.

10. Pinzette nach wenigstens einem der Ansprüche 1 bis 9, dadurch gekennzeichnet, daß die Rippen (32) der beiden Rinnenprofile (24 bzw. 24ₐ) mit fluchtenden Durchgängen (36) für Verbindungsglieder, insbesondere Schrauben (42), versehen sind, wobei gegebenenfalls die Durchgänge (36) eines Rinnenprofils (24, 24ₐ) Schraublöcher sind oder jeweils in einer an der Rinnenbodenaußenseite angeordnete Erweiterung (38) zur Aufnahme eines Schraubenkopfes (40) münden.

11. Pinzette nach wenigstens einem der Ansprüche 1 bis 10, dadurch gekennzeichnet, daß die innere Länge der Rinnenschenkel (28) kürzer ist als die halbe Breite (b₁) des Griffblattes (20) des Pinzettenarmes (12).

12. Pinzette nach wenigstens einem der Ansprüche 1 bis 11, dadurch gekennzeichnet, daß die Breite (b₁) des Griffblattes (20) unter Bildung schulterartiger Absätze (21) im Pinzettenarm (12) kürzer ist als dessen anschließende Breite (b), wobei gegebenenfalls die schulterartigen Absätze (21) Anschläge für die Rinnenprofile (24) sind.

13. Pinzette nach einem der Ansprüche 1 bis 12, dadurch gekennzeichnet, daß das Rinnenprofil (24) aus einer Leichtmetallegierung geformt und mit einer Eloxalschicht überzogen ist, wobei gegebenenfalls das Rinnenprofil (24) auf dem Wege des Strangpressens hergestellt und die Rippe (32) abgelängt ist.

14. Pinzette nach Anspruch 6, dadurch gekennzeichnet, daß der Stromanschlußstecker (50) zwei Einschubschlitze (56) für die Verlängerungsblätter (46) und an diesen jeweils zumindest eine Kontaktader (58) od.dgl. als Partner für die Kontaktfläche (47) des Verlängerungsblattes aufweist, wobei gegebenenfalls der Stromanschlußstecker (50) und ein in ihm verlaufender Klemmblock (54) von rechteckigem Querschnitt sind und die Einschubschlitze (56) begrenzen, wobei der Klemmblock (54) die Kontaktadern (58) trägt.

15. Pinzette mit jeweils eine isolierende Ummantelung und eine aus dieser ragende Spitze aufweisenden Pinzettenarmen sowie mit elektrischen Anschlußstellen an den der Halterung nahen Enden der Pinzettenarme nach einem der Ansprüche 9 bis 13, dadurch gekennzeichnet, daß im Bereich des freien Kopfraumes (25) die einander zugewandten, freien Innenflächen der Griffblätter (20) der Pinzettenarme (12) eine von der isolierenden Ummantelung (18) freien Kontaktabschnitt aufweisen, an den ein Kontaktstift od.dgl. Kontaktelement eines in den freien Kopfraum einführbaren Stromanschlußsteckers anlegbar ist.

16. Pinzette nach Anspruch 15, dadurch gekennzeichnet, daß der Kontaktstift federnd ausgebildet ist und/oder der Kontaktstecker lösbar im Kopfraum (25) ruht.
